# EUROPEAN PATENT APPLICATION

(11) **EP 2 682 105 A1**
(43) Date of publication of application: **08.01.2014**
(21) Application number: 13175224.8
(22) Date of filing: 05.07.2013
(51) Int. Cl.: A61K 9/00, A61K 31/192

(54) **Orally-disintegrating formulations of dexketoprofen**

(30) Priority: 06.07.2012 TR 201207916; 12.07.2012 TR 201208169
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

The present invention relates to an orally-disintegrating pharmaceutical tablet formulation, comprising dexketoprofen or a pharmaceutically acceptable salt of dexketoprofen and at least one methacrylate polymer and stearic acid.

## Description

### Field of Invention

The present invention relates to a formulation comprising dexketoprofen or a pharmaceutically acceptable salt of dexketoprofen. The present invention more particularly relates to an orally-disintegrating tablet formulation of dexketoprofen, which is stable against ambient influences, and has improved taste properties.

### Background of Invention

Dexketoprofen is a novel medicament with analgesic, anti-inflammatory, and antipyretic effects, belonging to the class of non-steroidal anti-inflammatory drugs. Its chemical designation is (2S)-2-[3-(benzoyl)phenyl]propanoic acid, with the chemical structure illustrated below in Formula 1.

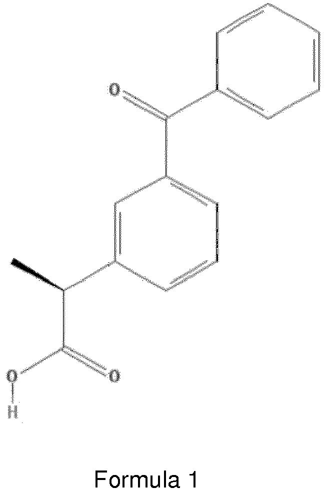

The dexketoprofen trometamol salt is marketed under the trademark Keral^{®} in 25 mg dosage forms.

There are various patents in the patent literature in relation to dexketoprofen.

The patent US3641127 discloses ketoprofen and pharmaceutically acceptable nontoxic salts thereof.

The patent application EP00996437 discloses a process for preparing a semisolid pharmaceutical formulation comprising dexketoprofen trometamol, a jelling agent and a pharmaceutically acceptable amine as a neutralizing agent.

The patent EP1154766 claims the use of dexketoprofen or derivatives thereof for producing medicaments for treating diseases which are positively influenced by the inhibition of NF-κB activation. These diseases may be rheumatic diseases, asthma, shock, inflammatory bowel disorders.

The taste of dexketoprofen has an unpleasant character in orally-disintegrating tablets and should be masked. However, when the unpleasant taste of dexketoprofen is masked, the stability and solubility values of dexketoprofen should be kept at desired levels.

Dexketoprofen is already presented in a conventional tablet formulation. This active pharmaceutical agent used for treating many diseases, however, is preferred in an orally-disintegrating form. These preparations are advantageous for use in elder patients with swallowing difficulty or in noncompliant patients. Orally-disintegrating dosage forms are convenient when drinking water is not available or not preferable.

Developing orally-disintegrating formulations is difficult due to several different reasons. First of all, the time required for the disintegration of the dosage form in the oral cavity under the presence of saliva is much shorter than the time required by the stomach. Therefore, these formulations are quite porous and therefore not too hard. Such porous formulations are prone to be susceptible to humidity. As a result of this, they lead to some stability problems. In addition, measures must be taken while preparing, packaging, handling and storing finished dosage forms of orally-disintegrating formulations.

For this reason, orally-disintegrating compositions of dexketoprofen or a pharmaceutically acceptable salt thereof is desired, which would overcome the drawbacks referred to hereinabove and would let the active agent be disintegrated and dissolved in the oral cavity.

In addition to the stability problems which may arise from the porous structure of this dosage form, other stability problems are frequently encountered in dexketoprofen formulations under the influence of ambiance and physical conditions. Dexketoprofen is an active agent that is highly-susceptible to air and humidity conditions. When dexketoprofen is initially exposed to air and humidity, it degrades structurally and develops chemical behavioral changes. As a result of this, two main problems emerge. The first problem is that the stability of the products developed is not at a desired level and the shelf life thereof is shortened. Secondly, dexketoprofen is reactive against the excipients employed in developing the formulations containing the same. This, in turn, causes impurities and unwanted components to be included into the formulation.

The choice of lubricants is extremely important for orally-disintegrating formulations. Whilst it is a frequently preferred lubricant, magnesium stearate has some known drawbacks, and therefore it is used in small amounts in the production of drugs. Magnesium stearate is hardly soluble in water, and due to this hydrophobic character, it may retard the dissolution of an active agent from a solid dosage form such as a tablet or capsule. The dissolution of a tablet is sensitive to both the amount of magnesium stearate present in the formulation and the blending time thereof. The blending time should be limited. Long blending times may result in the formation of hydrophobic powder beds which do not disperse easily and overblending can cause compaction problems. Tablet dissolution rate and crushing strength decrease as the time of blending increases; and magnesium stearate may also increase tablet friability. Blending times with magnesium stearate should therefore be carefully controlled. (Handbook of Pharmaceutical Excipients, 5th Edition, Rowe, Raymond C., Sheskey, Paul J., Owen, Sian C., pages 430-432).

Accordingly, there is a need for orally-disintegrating compositions of dexketoprofen or pharmaceutically-acceptable salts thereof, which would overcome the aforesaid drawbacks, would ensure a rapid disintegration and dissolution of the active agent in the oral cavity, would leave a pleasant sensation in the mouth, and would have a high mechanical strength adequate for processing in high-speed tablet compression machines and for transporting in low-cost packages, whereas the present invention discloses such formulations which would fulfill these requirements.

### Object and Brief Description of Invention

The present invention provides an easily administrable dexketoprofen formulation, eliminating all problems referred to above and bringing additional advantages to the relevant prior art.

Accordingly, the main object of the present invention is to obtain a novel orally-disintegrating formulation for use in the treatment of pain.

A further object of the present invention is to obtain a stable orally-administered dexketoprofen formulation with high bioavailability and with improved taste properties.

Another object of the present invention is to obtain a stable dexketoprofen formulation with high bioavailability.

A further object of the present invention is to provide a high dissolution rate for the stable dexketoprofen formulation with high bioavailability.

Another object of the present invention is to avoid any aggregation of ingredients of the composition and to provide a desired level of flowability during production by means of convenient excipients used while the formulation is obtained.

An orally-disintegrating pharmaceutical formulation has been developed to carry out any objects, referred to above and to emerge from the following detailed description.

In a preferred embodiment of the present invention, said novelty is carried out using dexketoprofen or a pharmaceutically acceptable salt of dexketoprofen, together with at least one methacrylate polymer and stearic acid. The formulation disintegrates in the oral cavity in 90 seconds and preferably in 40 seconds.

In a preferred embodiment of the present invention, said dexketoprofen is in the form of a trometamol salt thereof.

Another preferred embodiment of the present invention further comprises at least one excipient.

In another preferred embodiment of the present invention, said excipients are selected from the group consisting of disintegrants, diluents, binders, glidants, lubricants, sweeteners, flavoring agents, preserving agents and coloring agents.

In a preferred embodiment of the present invention, said lubricant is preferably sodium stearyl fumarate.

In a further preferred embodiment of the present invention, the proportion of dexketoprofen to the total amount of disintegrant is between 2 to 35, preferably 4 to 33, and more preferably 5 to 30.

In another preferred embodiment of the present invention, the proportion of the amount of said disintegrant in the inner phase to the amount thereof in the outer phase is in the range of 0,02 to 40.

In a further preferred embodiment of the present invention, said disintegrant is selected from the group consisting of at least one or a mixture of polyvinylpyrrolidone, croscarmellose sodium, crospovidone, low-substituted hydroxypropyl cellulose, hydroxypropyl methylcellulose, sodium starch glycolate, alginate or alginate salts, carboxymethyl cellulose calcium, carboxymethyl cellulose sodium, corn starch, pregelatinized starch, methyl cellulose, microcrystalline cellulose, soy polysaccharides.

In another preferred embodiment of the present invention, said disintegrant is preferably polyvinylpyrrolidone.

In another preferred embodiment of the present invention, said disintegrant is preferably croscarmellose sodium.

In another preferred embodiment of the present invention, said disintegrant is preferably sodium carboxymethyl cellulose and/or calcium carboxymethyl cellulose.

In another preferred embodiment of the present invention, said disintegrant is preferably low-substituted hydroxypropyl cellulose.

In another preferred embodiment of the present invention, said disintegrant is preferably sodium starch glycolate.

In another preferred embodiment of the present invention, said disintegrant is preferably alginate or salts of alginate.

In another preferred embodiment according to the present invention, said diluent is preferably mannitol and/or microcrystalline cellulose.

The methacrylate polymer is poly(butyl methacrylate-co-(2-dimethylaminoethyl) methacrylate-co-methyl methacrylate) 1:2:1.

In a preferred embodiment of the present invention, said lubricant is sodium stearyl fumarate.

In a further preferred embodiment of the present invention, said glidant is preferably colloidal silicon dioxide.

In another preferred embodiment of the present invention, said sweeteners are preferably sucralose, aspartame and/or saccharine.

In a further preferred embodiment of the present invention, said flavoring agents are preferably menthol and/or fruit extracts.

In another preferred embodiment of the present invention, said tablet is compressed under a force of 10 to 40 kN, preferably 15 - 35 kN, and more preferably 15 to 30 kN.

In a further preferred embodiment of the present invention, said formulation is in the form of a tablet.

A method for preparing a pharmaceutical formulation according to any of the preceding claims, this method comprising the steps of
a. weighing and mixing together dexketoprofen trometamol, fine grain mannitol, microcrystalline cellulose, disintegrant or some amount of the disintegrant,
b. preparing a solution of poly(butyl methacrylate-co-(2-dimethylaminoethyl) methacrylate-co-methyl methacrylate) 1:2:1, stearic acid in water,
c. adding this solution into the first mixture to give wet granules and drying and sieving the same,
d. then, sieving coarse grain mannitol and the remaining disintegrant, colloidal silicon dioxide, sucralose, mint flavor or menthol and orange flavor and adding into the granules,
e. sieving and adding sodium stearyl fumarate into the final mixture,
f. compressing the resulting mixture into tablets.

### Detailed Description of Invention

The present invention is described in more details with the following examples. These examples are not limiting the scope of the present invention and is to be considered under the light of the foregoing detailed disclosure. It is obvious that those skilled in the relevant art can produce similar embodiments under the light of the foregoing disclosures by making many adaptations to both the materials and the methods, without departing from the scope of the present invention.

This orally-disintegrating tablet composition composed of the followings is designed to minimize the disintegration time of tablets and maximize the mechanical strength.

### Example 1

| Ingredients | (w/w) % |
|---|---|
| dexketoprofen trometamol (25 mg equivalent) | 5 - 20 |
| microcrystalline cellulose | 1 - 10 |
| Mannitol 60 (fine grain) | 20 - 80 |
| sucralose | 1 - 3 |
| polyvinylpyrrolidone (inner phase) | 1 - 20 |
| polyvinylpyrrolidone (outer phase) | 1 - 20 |
| mint or menthol flavor | 1 - 10 |
| Mannitol DC 400 (coarse grain) | 1 - 20 |
| orange flavor | 0,01 - 5 |
| sodium stearil fumarat | 0,2 - 5 |
| colloidal silicon dioxide | 0,01 - 2 |
| poly(butyl methacrylate-co-(2-dimethylaminoethyl) methacrylate-co-methyl methacrylate) 1:2:1 | 1 - 5 |
| stearic acid | 1 - 3 |

Dexketoprofen trometamol, fine grain mannitol, microcrystalline cellulose, and some amount of polyvinylpyrrolidone are weighed and mixed. A solution of poly(butyl methacrylate-co-(2-dimethylaminoethyl) methacrylate-co-methyl methacrylate) 1:2:1, stearic acid is prepared in water. This solution is added into the first mixture to give wet granules, then these granules are dried in a drying oven and sieved. Then, coarse grain mannitol, the remaining polyvinylpyrrolidone, colloidal silicon dioxide, sucralose, mint or menthol flavor, and orange flavor are weighed, sieved and added into the previous mixture. Finally, based on yield calculations, sodium stearyl fumarate is weighed, sieved and added into the final mixture and mixed together. Thereafter, the mixture is compressed into tablets.

### Example 2

| Ingredients | (w/w) % |
|---|---|
| dexketoprofen trometamol (25 mg equivalent) | 5 - 20 |
| microcrystalline cellulose | 1 - 10 |
| Mannitol 60 (fine grain) | 20 - 80 |
| sucralose | 1 - 3 |
| sodium starch glycolate (inner phase) | 1 - 20 |
| sodium starch glycolate (outer phase) | 1 - 20 |
| mint or menthol flavor | 1 - 10 |
| Mannitol DC 400 (coarse grain) | 1 - 20 |
| orange flavor | 0,01 - 5 |
| sodium stearil fumarat | 0,2 - 5 |
| colloidal silicon dioxide | 0,01 - 2 |
| poly(butyl methacrylate-co-(2-dimethylaminoethyl) methacrylate-co-methyl methacrylate) 1:2:1 | 1 - 5 |
| stearic acid | 1 - 3 |

Dexketoprofen trometamol, fine grain mannitol, microcrystalline cellulose, and some amount of sodium starch glycolate are weighed and mixed. A solution of poly(butyl methacrylate-co-(2-dimethylaminoethyl) methacrylate-co-methyl methacrylate) 1:2:1, stearic acid is prepared in water. This solution is added into the first mixture to give wet granules, then these granules are dried in a drying oven and sieved. Then, coarse grain mannitol, the remaining amount of sodium starch glycolate, colloidal silicon dioxide, sucralose, mint or menthol flavor, and orange flavor are weighed, sieved and added into the previous mixture. Finally, based on yield calculations, sodium stearyl fumarate is weighed, sieved and added into the final mixture and mixed together. Thereafter, the mixture is compressed into tablets.

### Example 3

| Ingredients | (w/w) % |
|---|---|
| dexketoprofen trometamol (25 mg equivalent) | 5 - 20 |
| microcrystalline cellulose | 1 - 10 |
| Mannitol 60 (fine grain) | 20 - 80 |
| sucralose | 1 - 3 |
| crospovidone (inner phase) | 1 - 20 |
| crospovidone (outer phase) | 1 - 20 |
| mint or menthol flavor | 1 - 10 |
| Mannitol DC 400 (coarse grain) | 1 - 20 |
| orange flavor | 0,01 - 5 |
| sodium stearil fumarat | 0,2 - 5 |
| colloidal silicon dioxide | 0,01 - 2 |
| poly(butyl methacrylate-co-(2-dimethylaminoethyl) methacrylate-co-methyl methacrylate) 1:2:1 | 1 - 5 |
| stearic acid | 1 - 3 |

Dexketoprofen trometamol, fine grain mannitol, microcrystalline cellulose, and some amount of crospovidone are weighed and mixed. A solution of poly(butyl methacrylate-co-(2-dimethylaminoethyl) methacrylate-co-methyl methacrylate) 1:2:1, stearic acid is prepared in water. This solution is added into the first mixture to give wet granules, then these granules are dried in a drying oven and sieved. Then, coarse grain mannitol, the remaining crospovidone, colloidal silicon dioxide, sucralose, mint or menthol flavor, and orange flavor are weighed, sieved and added into the previous mixture. Finally, based on yield calculations, sodium stearyl fumarate is weighed, sieved and added into the final mixture and mixed together. Thereafter, the mixture is compressed into tablets.

### Example 4

| Ingredients | (w/w) % |
|---|---|
| dexketoprofen trometamol (25 mg equivalent) | 5 - 20 |
| microcrystalline cellulose | 1 - 10 |
| Mannitol 60 (fine grain) | 20 - 80 |
| sucralose | 1 - 3 |
| low-substituted hydroxypropyl cellulose (inner phase) | 1 - 20 |
| low-substituted hydroxypropyl cellulose (outer phase) | 1 - 20 |
| mint or menthol flavor | 1 - 10 |
| Mannitol DC 400 (coarse grain) | 1 - 20 |
| orange flavor | 0,01 - 5 |
| sodium stearil fumarat | 0,2 - 5 |
| colloidal silicon dioxide | 0,01 - 2 |
| poly(butyl methacrylate-co-(2-dimethylaminoethyl) methacrylate-co-methyl methacrylate) 1:2:1 | 1 - 5 |
| stearic acid | 1 - 3 |

Dexketoprofen trometamol, fine grain mannitol, microcrystalline cellulose, and some amount of low-substituted hydroxypropyl cellulose are weighed and mixed. A solution of poly(butyl methacrylate-co-(2-dimethylaminoethyl) methacrylate-co-methyl methacrylate) 1:2:1, stearic acid is prepared in water. This solution is added into the first mixture to give wet granules, then these granules are dried in a drying oven and sieved. Then, coarse grain mannitol, the remaining low-substituted hydroxypropyl cellulose, colloidal silicon dioxide, sucralose, mint or menthol flavor, and orange flavor are weighed, sieved and added into the previously mixture. Finally, based on yield calculations, sodium stearyl fumarate is weighed, sieved and added into the final mixture and mixed together. Thereafter, the mixture is compressed into tablets. Here, low-substituted hydroxypropyl cellulose may be in the form of LH 11 or LH 21.

### Example 5

| Ingredients | (w/w) % |
|---|---|
| dexketoprofen trometamol (25 mg equivalent) | 5 - 20 |
| microcrystalline cellulose | 1 - 10 |
| Mannitol 60 (fine grain) | 20 - 80 |
| sucralose | 1 - 3 |
| sodium alginate and/or calcium alginate (inner phase) | 1 - 20 |
| sodium alginate and/or calcium alginate (outer phase) | 1 - 20 |
| mint or menthol flavor | 1 - 10 |
| Mannitol DC 400 (coarse grain) | 1 - 20 |
| orange flavor | 0,01 - 5 |
| sodium stearil fumarat | 0,2 - 5 |
| colloidal silicon dioxide | 0,01 - 2 |
| poly(butyl methacrylate-co-(2-dimethylaminoethyl) methacrylate-co-methyl methacrylate) 1:2:1 | 1 - 5 |
| stearic acid | 1 - 3 |

Dexketoprofen trometamol, fine grain mannitol, microcrystalline cellulose, and some amount of sodium alginate and/or calcium alginate are weighed and mixed. A solution of poly(butyl methacrylate-co-(2-dimethylaminoethyl) methacrylate-co-methyl methacrylate) 1:2:1, stearic acid is prepared in water. This solution is added into the first mixture to give wet granules, then these granules are dried in a drying oven and sieved. Then, coarse grain mannitol, the remaining amount of sodium alginate and/or calcium alginate, colloidal silicon dioxide, sucralose, mint or menthol flavor, and orange flavor are weighed, sieved and added into the previously mixture. Finally, based on yield calculations, sodium stearyl fumarate is weighed, sieved and added into the final mixture and mixed together. Thereafter, the mixture is compressed into tablets.

The orally-disintegrating formulations of the present invention can be prepared by techniques such as direct compression, dry granulation, wet granulation, etc. well known by those skilled in the art. Orally-disintegrating compositions according to the present invention can also be prepared by means of technologies such as spray drying, freeze drying, floss formation process, die casting, Zydis^{®} technology, Flashtab technology, OraSolv^{®} technology, DuraSolv^{®} technology, Wowtab™ (nonaqueous immediately-soluble tablet) technology and other similar technologies. Dexketoprofen or a pharmaceutically acceptable salt thereof and other excipients are preferably mixed together and then the resulting mixture is compressed to give tablets.

The orally-disintegrating pharmaceutical formulation obtained has surprisingly shown a quite good stability, solubility and dissolution rates. The excipients used bring the stability of the formulation to a desired level. Stability and the taste-masking effect is provided basically with poly(butyl methacrylate-co-(2-dimethylaminoethyl) methacrylate-co-methyl methacrylate) 1:2:1 and stearic acid. This polymer opening under pH 5.5 disintegrates in the mouth, but dissolves in the stomach and ensures its efficiency. The formulation is coated with methacrylate polymer and stearic acid for stability and taste masking purposes. Stearic acid provides for an efficient coating of the surface of granules. The sweetener and flavoring agents also contribute to the taste masking effect. Sodium stearil fumarate used in the formulation is a very efficient lubricant, being less hydrophobic than magnesium stearate and having a lower retarding effect, as compared to magnesium stearate, when the tablet is dissolved. Additionally, sodium stearyl fumarate increases the formulation's stability and does not give rise to overmixing-related problems, which is observed with magnesium stearate. These excipients also avoid the aggregation of particles or granules and any reduction in their flowability during production.

Two types of mannitol are used in the formulation. The particle size of the fine grain mannitol is 1 - 200 µm and facilitates disintegration. The particle size of the coarse grain mannitol is 150 - 700 µm and enhances the formulation's flowability and facilitates the compression of tablets. A composition of mannitol may be as following.

Mannitol (fine grain): d(0.1): 6 µm, d(0.5): 44 µm, d(0.9): 166 µm
Mannitol (coarse grain): d(0.1): 160 µm, d(0.5): 338 µm, d(0.9): 577 µm

Said tablet is compressed under a force of 10 to 40 kN, preferably 15-35 kN, and more preferably 15 to 30 kN. This, in turn, both relates to the stability of the tablet since the interior thereof contacts with air and humidity, and directly effects the dissolution rate.

The present invention is used in the prevention or treatment of pain, ankylosing spondylitis, arthralgia, fever, dysmenorrhoea, toothache, gouty arthritis, myalgia, osteoarthritis, rheumatoid arthritis in mammals, particularly in humans.

Orally-disintegrating formulations of the present invention comprise tablets, single-dose packages, mini tablets, and multilayered and multicoated tablets, pellets, or powders formulated according to the standard methods of the relevant art. The formulation is preferably in the form of tablets.

The orally-disintegrating tablet composition of the present invention is preferably in the form of a disk, circle, sphere, lokma, bar, polygon, ellipse, etc..

Pharmaceutical formulations of the present invention may also comprise one or more pharmaceutically acceptable excipient(s). Such pharmaceutically acceptable convenient excipients include, but are not restricted to fillers, glidants, lubricants, disintegrants, surface active agents etc. and the mixtures thereof.

Suitable binders include, but are not restricted to polyvinylpyrrolidone, polyethylene glycol, polyvinyl alcohol, hydroxypropyl methylcellulose, carboxymethyl cellulose, methyl cellulose and other cellulose derivatives, gelatin, polyvinyl alcohol, carrageenan, guar gum, xanthan gum, and methacrylate acid derivatives and the mixtures thereof.

Suitable lubricants include, but are not limited to magnesium stearat, calcium stearat, sodium stearyl fumarate, sodium lauryl sulfate, stearic acid, etc. and the mixtures thereof.

Suitable glidants include, but are not restricted to colloidal silicon dioxide, talk, aluminum silicate, etc., and the mixtures thereof.

The orally-disintegrating compositions according to the present invention can also comprise sweeteners and flavoring agents to enhance patient compliance.

Suitable sweeteners include, but are not restricted to aspartame, sucralose, saccharine; glucose, lactose, fructose and other sugars, and mannitol, sorbitol, xylitol, erythritol, and other sugar alcohols, etc., and the mixtures thereof.

Suitable flavoring agents include, but are not restricted to menthol, mint, cinnamon, chocolate, vanillin, and fruit extracts such as cherry, orange, strawberry, grape, and the mixtures thereof.

Suitable coloring agents include, but are not restricted to iron oxide, FD&C (Food, Drug and Cosmetic) dyes, ponceau, etc. and the mixtures thereof.

## Claims

1. An orally-disintegrating pharmaceutical tablet formulation, comprising dexketoprofen or a pharmaceutically acceptable salt of dexketoprofen and at least one methacrylate polymer and stearic acid.

2. The pharmaceutical formulation according to Claim 1, further comprising at least one excipient.

3. The pharmaceutical formulation according to any of the preceding claims, wherein said excipients are selected from the group consisting of disintegrants, diluents, binders, glidants, lubricants, sweeteners, flavoring agents, preserving agents and coloring agents.

4. The pharmaceutical formulation according to any of the preceding claims, wherein said lubricant is preferably sodium stearyl fumarate.

5. The pharmaceutical formulation according to any of the preceding claims, wherein the proportion of dexketoprofen to the total amount of disintegrant is in the range of 2 to 35, preferably in the range of 4 to 33, and more preferably in the range of 5 to 30.

6. The pharmaceutical formulation according to any of the preceding claims, wherein the proportion of the amount of said disintegrant in the inner phase to the amount thereof in the outer phase is in the range of 0,02 to 40.

7. The pharmaceutical formulation according to any of the preceding claims, wherein said disintegrant is selected from the group consisting of at least one or a mixture of polyvinylpyrrolidone, croscarmellose sodium, crospovidone, low-substituted hydroxypropyl cellulose, hydroxypropyl methylcellulose, sodium starch glycolate, alginate or alginate salts, carboxymethyl cellulose calcium, carboxymethyl cellulose sodium, corn starch, pregelatinized starch, methyl cellulose, microcrystalline cellulose, soy polysaccharides.

8. The pharmaceutical formulation according to any of the preceding claims, wherein said disintegrant is preferably polyvinylpyrrolidone.

9. The pharmaceutical formulation according to any of the preceding claims, wherein said disintegrant is preferably croscarmellose sodium.

10. The pharmaceutical formulation according to any of the preceding claims, wherein said disintegrant is preferably sodium carboxymethyl cellulose and/or calcium carboxymethyl cellulose.

11. The pharmaceutical formulation according to any of the preceding claims, wherein said disintegrant is preferably low-substituted hydroxypropyl cellulose.

12. The pharmaceutical formulation according to any of the preceding claims, wherein said disintegrant is preferably sodium starch glycolate.

13. The pharmaceutical formulation according to any of the preceding claims, wherein said disintegrant is preferably alginate or salts of alginate.

14. The pharmaceutical formulation according to any of the preceding claims, wherein said super disintegrant is preferably croscarmellose sodium.

15. The pharmaceutical formulation according to any of the preceding claims, wherein said diluents are preferably mannitol and/or microcrystalline cellulose.

16. The pharmaceutical formulation according to any of the preceding claims, wherein said methacrylate polymer is poly(butyl methacrylate-co-(2-dimethylaminoethyl) methacrylate-co-methyl methacrylate) 1:2:1.

17. The pharmaceutical formulation according to any of the preceding claims, wherein said lubricants are preferably magnesium stearate and sodium stearyl fumarate.

18. The pharmaceutical formulation according to any of the preceding claims, wherein said glidant is preferably colloidal silicon dioxide.

19. The pharmaceutical formulation according to any of the preceding claims, wherein said sweeteners are preferably aspartame, sucralose and/or saccharine.

20. The pharmaceutical formulation according to any of the preceding claims, wherein said flavoring agents are preferably mint flavor/menthol and/or fruit extracts.

21. The pharmaceutical composition according to any of the preceding claims, wherein said tablet is compressed under a force of 10 to 40 kN, preferably 15 - 35 kN, and more preferably under a force of 15 to 30 kN.

22. A method for preparing a pharmaceutical formulation according to any of the preceding claims, this method comprising the steps of
a. weighing and mixing together dexketoprofen trometamol, fine grain mannitol, microcrystalline cellulose, disintegrant or some amount of the disintegrant,
b. preparing a solution of poly(butyl methacrylate-co-(2-dimethylaminoethyl) methacrylate-co-methyl methacrylate) 1:2:1, stearic acid in water,
c. adding this solution into the first mixture to give wet granules and drying and sieving the same,
d. then, sieving coarse grain mannitol and the remaining disintegrant, colloidal silicon dioxide, sucralose, mint flavor or menthol and orange flavor and adding into the granules,
e. sieving and adding sodium stearyl fumarate into the final mixture,
f. compressing the resulting mixture into tablets.

23. A pharmaceutical formulation according to any of the preceding claims for use in the prevention or treatment of pain, ankylosing spondylitis, arthralgia, fever, dysmenorrhoea, toothache, gouty arthritis, myalgia, osteoarthritis, rheumatoid arthritis in mammals, particularly in humans.
